# EUROPEAN PATENT SPECIFICATION

(11) **EP 3 808 533 B1**
(45) Date of publication and mention of the grant of the patent: **06.09.2023**
(21) Application number: 19818682.7
(22) Date of filing: 14.06.2019
(51) Int. Cl.: B29C 48/09, B29C 48/12, B29C 48/91, B29C 48/18, A61M 25/10, B29L 31/00, B29C 48/151, B29C 48/21

(54) **METHOD FOR MANUFACTURING BALLOON CATHETER BY USING HEAT-CURABLE RESIN AND APPARATUS THEREFOR**
VERFAHREN ZUR HERSTELLUNG EINES BALLONKATHETERS UNTER VERWENDUNG VON WÄRMEHÄRTBAREM HARZ UND VORRICHTUNG DAFÜR
MÉTHODE DE FABRICATION D'UN CATHÉTER À BALLONNET À L'AIDE D'UNE RÉSINE THERMODURCISSABLE ET APPAREIL ASSOCIÉ

(30) Priority: 15.06.2018 KR 20180069193
(43) Date of publication of application: 21.04.2021
(73) Proprietor: Lee, Je-Kwon, Bucheon-si, Gyeonggi-do 14533 (KR)
(72) Inventor: Lee, Je-Kwon, Bucheon-si, Gyeonggi-do 14533 (KR)
(74) Representative: Contadin, Giorgio
(86) International application number: PCT/KR2019/007219
(87) International publication number: WO 2019/240542

(56) References cited:
- DE-A1-102015 220 571
- FR-A1- 2 673 110
- KR-A- 20050 071 453
- KR-A- 20140 012 683
- KR-B1- 100 333 264
- KR-B1- 100 530 607
- KR-B1- 101 527 340
- KR-B1- 101 666 944
- US-B1- 6 235 226

## Description

### [Field of Invention]

The present invention relates to a method of manufacturing a balloon catheter using a thermosetting resin, and more particularly to an economical method of being capable of very promptly manufacturing a balloon catheter using a thermosetting resin through a continuous process, in which a drying process and a curing process are combined, after release agent application using the curing characteristics of a thermosetting resin.

### [Background of Invention]

As is well known, a thermosetting resin refers to a low-molecular-weight resin that is crosslinked by heat, a catalyst, ultraviolet rays, etc., thereby being converted into a polymer with a three-dimensional network structure. A representative example thereof is silicone rubber.

Due to such characteristics, a thermosetting resin presents as an amorphous material when it is in a low-molecular-weight resin state before curing, thereby being deformed by external force. However, after being converted into a polymer material due to crosslinking reaction due to heat or the like, the thermosetting resin becomes insoluble or non-meltable and is solidified.

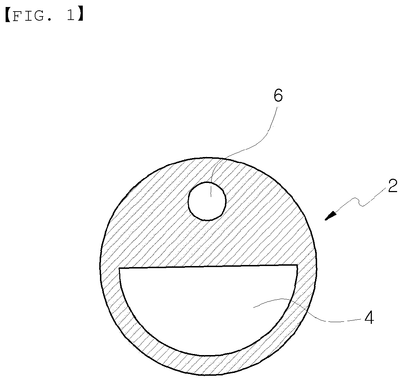

Meanwhile, a balloon catheter 2, which is a tube-shaped medical device, is inserted into a human organ, and then a balloon is inflated and fixed using an inflation lumen 6, and then, through a drainage lumen 4 or a drug lumen, a drug is injected or various liquid waste is removed. As shown in FIG. 1, a main body of the balloon catheter 2 is generally composed of an extruded tube having a 2-way structure wherein the inflation lumen 6 for inflation or contraction of a balloon and the drainage lumen 4 for drug injection or liquid waste removal are extruded as a single tube.

The extruded tube is manufactured by extruding a soft synthetic resin through an extruder. A predetermined part of the inflation lumen of the extruded tube is perforated, and a separately manufactured balloon is assembled. The balloon is completed through adhesion and coating. A separate hole is formed at the drainage lumen for drug injection or liquid waste removal. Accordingly, a main part of a catheter is completed.

A process of the catheter manufacturing method is complicated, and the manufacturing cost thereof is high. To address such problems, Korean Patent Nos. 10-0333264 and 10-0434720 have recently proposed methods of manufacturing a balloon catheter to which a technology capable of eliminating assembly, adhesion, and coating processes of a balloon is applied.

In the case of Korean Patent No. 10-0333264, a balloon is formed through a dipping process using liquid silicone, so that, if a catheter tube is not kept exactly perpendicular to the sea level until the balloon is cured, problems related to the symmetry of the balloon may occur. Accordingly, defects due to asymmetric balloons frequently occur, so that mass production is impossible unless the process is controlled very sensitively.

With regard to Korean Patent No. 10-0434720, an extruded tube having the cross-section illustrated in Fig. 1 is first extruded (hereinafter referred to as first extrusion) more thinly than the thickness of a final product, and then the first extruded tube is cut to an appropriate length, and then inflation lumen perforation, release agent application, release agent drying, and overlay extrusion are performed (hereinafter referred to as second extrusion) so as to produce a catheters including a balloon that is embedded in a tube. This method is simpler than existing methods because it excludes complicated processes related to a balloon. However, since the method requires inflation lumen perforation, release agent application, and drying processes, the first extruded tube must inevitably be cut and processed. In addition, the production cost thereof is still high because continuous production is not possible according to the intermittent work process.

Meanwhile, Korean Patent No. 10-0689238 proposes a continuous production method to supplement the disadvantages of Korean Patent No. 10-0434720. However, Patent Registration No. 10-0689238 has risks that soot remains on a perforated area upon perforation of an inflation lumen with a laser, a tube may catch fire during the manufacturing process, and a drying process after release agent application is not specified.

To compensate for such problems, Korean Patent Application No. 10-2017-89480 changes an internal shape to omit an inflation lumen perforation process, and completes a release agent drying process within a few seconds using a hot air concentration method. However, since this method is merely an automation of an existing manual processing process, first extrusion, release agent application, release agent drying and second extrusion are still necessary. In addition, since investment in automation equipment is required, there is a limit in reducing manufacturing cost.

In addition, Korean Patent Application No. 10-2017-37885 proposes a method of feeding a first extruded tube into a second extruder to perform overlay extrusion, wherein the position of an adhesion guide pipe or an adhesion prevention pipe is controlled at an end of an extruder die to continuously produce a balloon part and a body part of a tube by section. However, this method may not be suitable for securing the thickness of a balloon due to the thickness of the adhesion guide pipe or the adhesion prevention pipe itself.

For example, the thickness of a balloon varies depending on manufacturers of a catheter, but is generally in the range of 0.35 mm to 0.5 mm. In the case of a production method recommended in the invention, a first extruded tube is produced, and then is fed into a second extruder. Accordingly, after an outer diameter of the first extruded tube is determined, the first extruded tube is introduced into the second extruder. Next, since an adhesion prevention pipe repeatedly produces a balloon and a body while reciprocating between a second extruded material and the first extruded tube, an inner diameter of the adhesion prevention pipe should be at least larger than an outer diameter of the first extruded tube such that the first extruded tube is smoothly fed into the second extruder.

The adhesion prevention pipe having an inner diameter larger than an outer diameter of the first extrusion tube; and an outer layer covered with a soft material of a balloon due to the thickness of the adhesion prevention pipe should be extruded to have an empty space at least as much as the thickness of the adhesion prevention pipe. In addition, since a final thickness after second extrusion is determined by an external die of a second extruder and a final thickness of a planned catheter, it is impossible to change the final thickness.

Accordingly, the thickness of the balloon is formed to be as thin as the thickness of the adhesion prevention pipe. When the adhesion prevention pipe is too thin, the durability of the adhesion prevention pipe itself is not secured, so that mass production is impossible. When the adhesion prevention pipe is too thick, a balloon layer becomes thin, so that it is impossible to create a desired balloon capacity. To address this, a first tube that is initially fed should be manufactured as thinly as the thickness of the adhesion prevention pipe. However, this method also cannot be applied to a catheter that is thinner than a certain thickness because the first tube cannot be manufactured extremely thinly.

Finally, to address the problems of Korean Patent Application No. 10-2017-37885, Korean Patent Application No. 10-2017-0165637 proposes to extrude a first extruded tube together with a second extruded material through a variable die in a state in which the first extruded tube is not cured. By this method, the first and second extruded tubes pass through a variable die, which is similar to the adhesion prevention pipe, at the point where the first extruded tube is not cured, so that constraints on the thicknesses of the first extruded tube and the adhesion prevention pipe are addressed, so that the problems of Korean Patent Application No. 10-2017-37885 can be addressed. However, the first and second extruded materials contact each other if curing is not directly performed at an end of an extruder die upon formation of a balloon by the variable die, so that a balloon is not formed. Accordingly, it is not possible to increase an extrusion speed to a certain degree or higher for curing immediately upon ejection. This is directly connected to a production speed and causes unit costs to rise. French Patent Application FR 2 673 110 A1 discloses a method for producing a catheter, in particular a Foley catheter, intended to be introduced into a body channel and comprising an inflatable balloon in the vicinity of its distal end.

As described above, the existing technologies require high manufacturing costs due to complicated processes, and can be applied only in a limited way due to various constraints. Accordingly, there is an urgent need for improvement

### [Summary of Invention]

### [Technical Problem to be Solved]

Therefore, the present invention has been made in view of the above problems, and it is one object of the present invention to provide an economical method of being capable of very promptly manufacturing a balloon catheter using a thermosetting resin through a continuous process, in which a drying process and a curing process are combined, after release agent application using the curing characteristics of a thermosetting resin.

### [Technical Solution]

In accordance with an aspect of the present invention, the above and other objects can be accomplished by the provision of a method of manufacturing a balloon catheter using a thermosetting resin, the method including: a) a process of extruding a first tube 14, on an outer periphery of which an inflation lumen 16 is formed in a longitudinal direction; b) a process of spraying a release agent in a non-contact manner to alternately apply the release agent to predetermined sections on an outer periphery of the first tube 14; c) a process of thermally curing a resin and drying the release agent using a heater; and d) a process of coating an outside of the first tube 14 with a balloon material to manufacture a balloon catheter tube 50, in which a balloon is repeatedly formed, in a longitudinal direction.

Preferably, process b) may further include a process of dividing a spray region and a non-spray region by a spray restriction film 26 upon spray of the release agent.

Preferably, the method may further include a process of periodically sucking a residual of the sprayed release agent through suction holes 34 formed in the spray restriction film 26.

Preferably, the method may further include a process of installing a heating wire at the spray restriction film 26 to evaporate an accumulated residual of the release agent.

In accordance with another aspect of the present invention, there is provided a device for manufacturing a balloon catheter using a thermosetting resin, the device includes: a first extruder 20 configured to extrude a thermosetting resin using a tube manufacturing mold 8 to manufacture a first tube 14; a release agent spraying device 22 disposed at a rear end of the tube manufacturing mold 8 and configured to alternately apply a release agent to a predetermined part on an outer periphery of the first tube 14; a first heater 38 configured to cure and dry the first tube 14 that has passed through the release agent spraying device 22, and the coated release agent; a second extruder 40 configured to extrude and cover a coating layer 46 on the outer periphery of the first tube 14, which has passed through the first heater 38, using a coating mold 41; and a second heater 42 configured to cure the first tube 14 on which the coating layer has been formed.

Preferably, the release agent spraying device 22 may include: a nozzle 24 for spraying a release agent; and a spray restriction film 26 bent to limit a spraying range of a release agent that is sprayed through the nozzle 24.

Preferably, the spray restriction film 26 may further include a release agent residual collection device 30, the release agent residual collection device 30 including: a suction pipe 32, inside of which a pipe conduit is formed; a plurality of suction holes 34 formed on inner side surfaces of the suction pipe 32 to suck a residual amount of a release agent; and a hose 36 connected to a vacuum pump so as to apply negative pressure to the suction pipe 32.

Preferably, the spray restriction film 26 may further include a heating means for evaporating a residual release agent accumulated on a predetermined part of the spray restriction film 26.

### [Effect of Invention]

As apparent from the above description, a method of manufacturing a balloon catheter using a thermosetting resin according to the present invention is characterized by curing a first tube and, immediately, feeding the first tube into a second extruder to complete a balloon catheter tube. Accordingly, manufacturing time and cost can be saved, compared to methods requiring a separate process. In addition, compared to methods using a variable die, etc., rapid production is possible. Further, since there is no waiting time in the process, defects due to contamination can be prevented.

### [Brief Description of Drawings]

FIG. 1 illustrates a cross-sectional view of an existing balloon catheter tube having a 2-way structure.

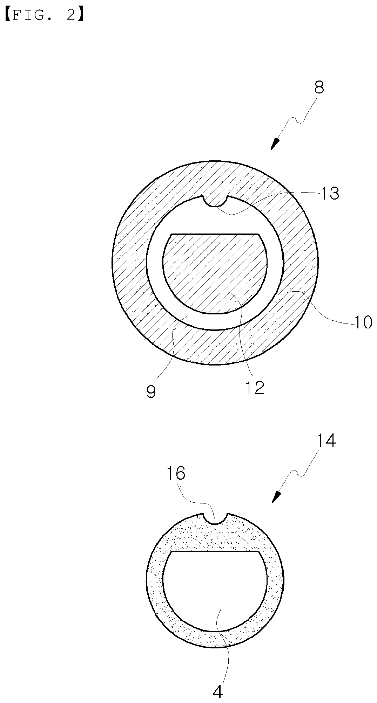

FIG. 2 illustrates a cross-sectional view of a first extruded tube according to an embodiment of the present invention.

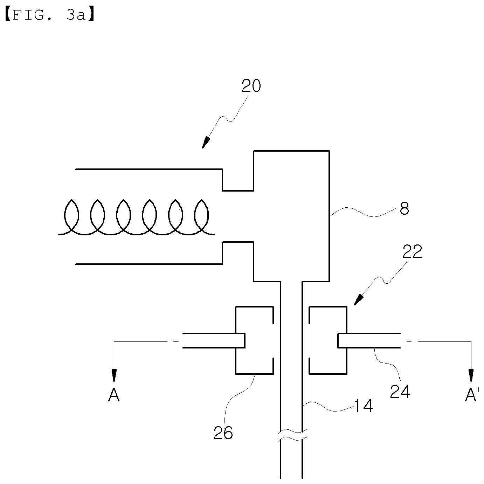

FIG. 3a illustrates a spray application process according to an embodiment of the present invention to which a spray restriction film is applied.

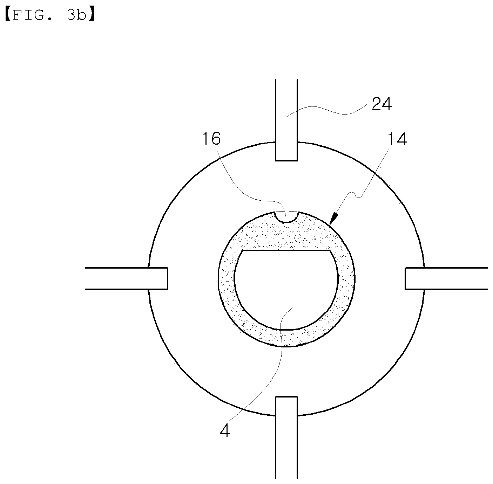

FIG. 3b illustrates the structures of a release agent coating device and a spray restriction film.

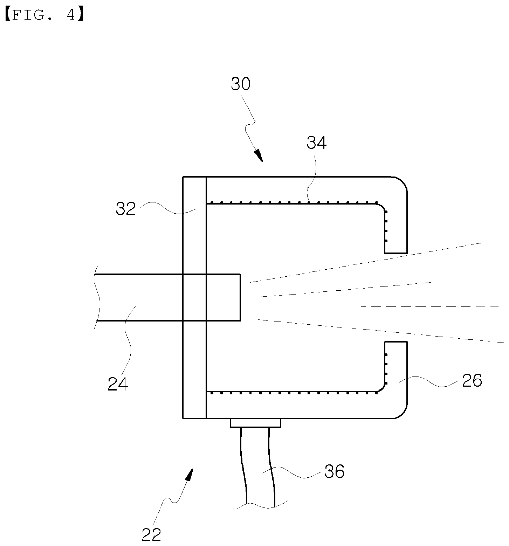

FIG. 4 illustrates a release agent residual collection device according to an embodiment of the present invention.
FIG. 5 illustrates a sectional view of a die used in a second extrusion process according to an embodiment of the present invention and a manufacturing state according to the second extrusion process.
FIG. 6 illustrates a sectional view of each section of a tube according to an embodiment of the present invention in which second extrusion is completed.
FIG. 7 illustrates a structural diagram of an entire facility according to an embodiment of the present invention.

### [Best Mode]

Accordingly, when negative pressure is periodically applied to a suction pipe 32 through a suction means such as a compressor (not shown), a residual release agent that flows down inner walls of the spray restriction film 26 may be sucked through the suction holes 34.

In accordance with an embodiment of the present invention, provided is a method of heating the spray restriction film 26 itself to immediately evaporate a sprayed release agent accumulated on the spray restriction film 26. That is, when a heating wire (not shown) is installed at a predetermined part of the spray restriction film 26 and is heated to the boiling point of the release agent or to a higher temperature, the residual of the sprayed release agent evaporates immediately upon contact with the spray restriction film 26, so that subsequent defects may be prevented.

In addition, since the first tube 14 may be cured to some extent by heat of the spray restriction film 26 according to the method, burden on the first heater 38 used after application with the release agent may be reduced.

A spray coating process with the release agent is performed in a manner of spraying and coating every predetermined tube length in consideration of an extrusion speed of the tube, or in a manner of spraying and coating at regular time intervals.

Next, the non-cured tube coated with the release agent at regular intervals is fed into the first heater 38. The first heater 38 is generally at about 600°C, and heats the tube in a manner of radiation and convection without contacting the tube to cause crosslinking reaction of the thermosetting material to be cured. Here, the sprayed and coated release agent is also dried by sufficient heat.

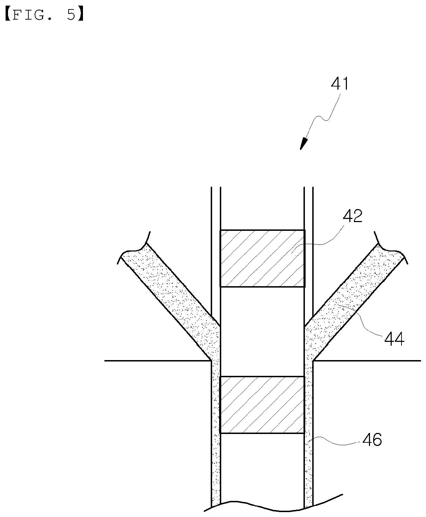

The cured first tube 14, the release agent on which has been dried, is fed into a second extruder 40 having the structure of FIG. 5 and is coated with a balloon material. Since curing of the first tube 14 and drying of the release agent thereon have been completed by the previous process, the first tube 14 may be directly fed into the second extruder 40 without separate processing to produce a balloon catheter tube 50.

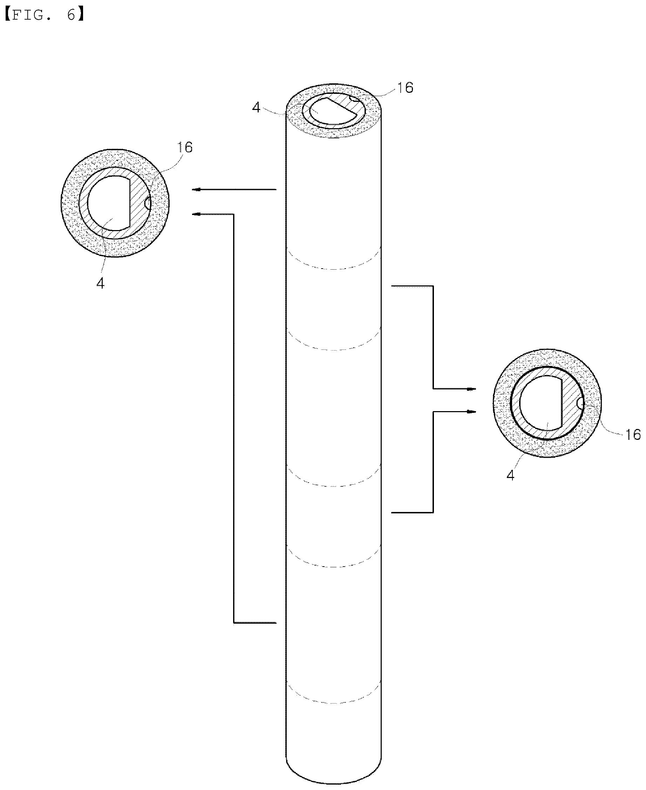

Since a balloon material of the coating layer 46 which is coated and formed in the second extruder 40 is also a thermosetting resin, a second extrusion material is cured through another second heater 42 after the second extruder 40. This structure is similar to the structure of a heater 38 located at a first extruder 20, and a balloon material is cured in a non-contact state to complete a tube wherein balloon catheter tubes having the shape shown in FIG. 6 are repeatedly connected. This tube is appropriately cut in consideration of release agent application sections set above, so that a balloon catheter tube is finally completed.

If setting of an appropriate length to be cut causes an error due to the elasticity of the balloon catheter tube 50 itself and, accordingly, it is impossible to stably produce a good product, sections coated with a release agent may be cut while observing with a camera, or, for more reliable classification, an ink spraying process together with the spray coating process with a release agent may be performed to continuously produce tubes, and then detect and cut ink-applied portions on the tubes, and discard ink-printed parts.

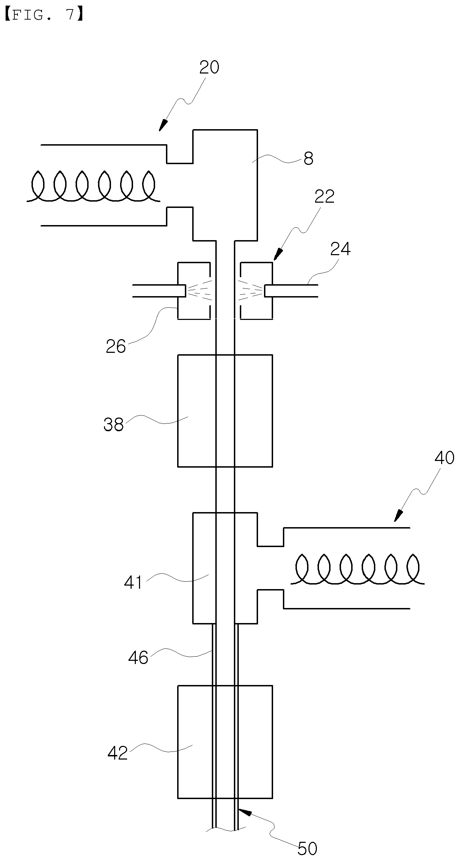

Finally, in accordance with a preferred embodiment of the present invention, the tube 50 of the balloon catheter may be rapidly, simply manufactured using the aforementioned preferred facility shown in FIG. 7.

Meanwhile, a method of manufacturing a balloon catheter using a thermosetting resin according to an embodiment of the present invention is not limited to the aforementioned embodiment, and may be modified in various forms without departing from the technical gist of the present invention.

### *Explanation of symbols*

8: tube manufacturing mold, 9: hollow,
10: external mold, 12: discharge tube formation mold,
[13: inflation lumen formation protrusion, 14: first tube,
20: first extruder, 22: release agent coating device,
24: nozzle, 26: spray restriction film,
30: release agent residual collection device, 32: suction pipe,
34: suction hole, 36: hose,
38: first heater, 40: second extruder,
41: coating mold, 42: second heater,
50: balloon catheter tube.

## Claims

1. A device for manufacturing a balloon catheter using a thermosetting resin, the device comprising:
a first extruder (20) configured to extrude a thermosetting resin using a tube manufacturing mold (8) to manufacture a first tube (14);
a release agent spraying device (22) disposed at a rear end of the tube manufacturing mold (8) and configured to alternately apply a release agent to a predetermined part on an outer periphery of the first tube (14);
a first heater (38) configured to cure and dry the first tube (14) that has passed through the release agent spraying device (22), and the coated release agent;
a second extruder (40) configured to extrude and cover a coating layer (46) on the outer periphery of the first tube (14), which has passed through the first heater (38), using a coating mold (41); and
a second heater (42) configured to cure the first tube (14) on which the coating layer has been formed,
wherein the release agent spraying device (22) comprises:
a nozzle (24) for spraying a release agent; and
a spray restriction film (26) bent to limit a spraying range of a release agent that is sprayed through the nozzle (24) .

2. The device according to claim 1, wherein the spray restriction film (26) further comprises a release agent residual collection device (30), the release agent residual collection device (30) comprising:
a suction pipe (32), inside of which a pipe conduit is formed;
a plurality of suction holes (34) formed on inner side surfaces of the suction pipe (32) to suck a residual amount of a release agent; and
a hose (36) connected to a vacuum pump so as to apply negative pressure to the suction pipe (32).

3. The device according to claim 1, wherein the spray restriction film (26) further comprises a heating means for evaporating a residual release agent accumulated on a predetermined part of the spray restriction film (26).

4. A method of manufacturing a balloon catheter using a thermosetting resin and the device according to one or more of the preceding claims, the method comprising:
a) extruding the thermosetting resin to make a first tube (14), on an outer periphery of which an inflation lumen (16) is formed in a longitudinal direction, wherein a spray region and a non-spray region are divided by a spray restriction film (26) upon spray of the release agent;
b) a process of spraying a release agent in a non-contact manner to alternately apply the release agent to predetermined sections on the outer periphery of the first tube (14);
c) thermally curing [[a]] the resin and drying the release agent using a heater; and
d) coating an outside of the first tube (14) with a balloon material to manufacture a balloon catheter tube (50), in which a balloon is repeatedly formed, in a longitudinal direction.

5. The method according to claim 4, further comprising: periodically sucking a residual of the sprayed release agent through suction holes (34) formed in the spray restriction film (26).

6. The method according to claim 4, further comprising: installing a heating wire at the spray restriction film (26) to evaporate an accumulated residual of the release agent.

## Patentansprüche

1. Vorrichtung zur Herstellung eines Ballonkatheters unter Verwendung eines wärmehärtenden Harzes, wobei die Vorrichtung umfasst:
einen ersten Extruder (20), der so konfiguriert ist, dass er ein wärmehärtendes Harz unter Verwendung einer Röhrenherstellungsform (8) extrudiert, um eine erste Röhre (14) herzustellen;
eine Trennmittel-Sprühvorrichtung (22), die an einem rückwärtigen Ende der Röhrenherstellungsform (8) angeordnet und so konfiguriert ist, dass sie alternierend ein Trennmittel auf ein vorbestimmtes Teil an einem Außenumfang der ersten Röhre (14) aufbringt;
eine erste Heizung (38), die so konfiguriert ist, dass sie die erste Röhre (14), die die Trennmittel-Sprühvorrichtung (22) durchlaufen hat, und das beschichtete Trennmittel härtet und trocknet;
einen zweiten Extruder (40), der so konfiguriert ist, dass er unter Verwendung einer Beschichtungsform (41) eine Beschichtungsschicht (46) auf den Außenumfang der ersten Röhre (14), die die erste Heizung (38) durchlaufen hat, extrudiert und bedeckt; und
eine zweite Heizung (42), die so konfiguriert ist, dass sie die erste Röhre (14), auf der die Beschichtungsschichtgebildet wurde, härtet,
wobei die Trennmittel-Sprühvorrichtung (22) umfasst:
eine Düse (24) zum Sprühen eines Trennmittels; und
einen Sprühbegrenzungsfilm (26), der so gebogen ist, dass er einen Sprühbereich eines Trennmittels, das durch die Düse (24) gesprüht wird, begrenzt.

2. Vorrichtung nach Anspruch 1, wobei der Sprühbegrenzungsfilm (26) ferner eine Vorrichtung (30) zum Sammeln von Trennmittelrückständen umfasst, wobei die Vorrichtung (30) zum Sammeln von Trennmittelrückständen umfasst:
ein Saugrohr (32), in dem eine Rohrleitung geformt ist;
eine Vielzahl von Sauglöchern (34), die an inneren Seitenflächen des Saugrohrs (32) ausgebildet sind, um eine Restmenge eines Trennmittels anzusaugen; und
einen Schlauch (36), der mit einer Vakuumpumpe verbunden ist, um einen Unterdruck auf das Saugrohr (32) auszuüben.

3. Vorrichtung nach Anspruch 1, wobei der Sprühbegrenzungsfilm (26) ferner ein Heizmittel zum Verdampfen eines restlichen Trennmittels umfasst, das sich auf einem vorbestimmten Teil des Sprühbegrenzungsfilms (26) angesammelt hat.

4. Verfahren zur Herstellung eines Ballonkatheters unter Verwendung eines wärmehärtenden Harzes und der Vorrichtung nach einem der vorhergehenden Ansprüche, wobei das Verfahren umfasst:
a) Extrudieren des wärmehärtenden Harzes, um eine erste Röhre (14) herzustellen, an deren Außenumfang ein Aufblaslumen (16) in einer Längsrichtung gebildet wird, wobei ein Sprühbereich und ein Nicht-Sprühbereich durch einen Sprühbegrenzungsfilm (26) beim Sprühen des Trennmittels getrennt werden;
b) ein Verfahren, bei dem ein Trennmittel berührungslos aufgesprüht wird, um das Trennmittel alternierend auf vorbestimmte Abschnitte des Außenumfangs der ersten Röhre (14) aufzubringen;
c) thermisches Härten [[a]] des Harzes und Trocknung des Trennmittels mit Hilfe einer Heizung; und
d) Beschichten einer Außenseite der ersten Röhre (14) mit einem Ballonmaterial zur Herstellung einer Ballonkatheterröhre (50), in dem wiederholt ein Ballon in einer Längsrichtung gebildet wird.

5. Verfahren nach Anspruch 4, ferner umfassend:
periodisches Saugen eines Restes des gesprühten Trennmittels durch Sauglöcher (34), die in dem Sprühbegrenzungsfilm (26) ausgebildet sind.

6. Verfahren nach Anspruch 4, ferner umfassend:
Anbringen eines Heizdrahtes an dem Sprühbegrenzungsfilm (26), um einen angesammelten Rest des Trennmittels zu verdampfen.

## Revendications

1. Dispositif de fabrication d'un cathéter à ballonnet à l'aide d'une résine thermodurcissable, le dispositif comprenant :
une première extrudeuse (20) configurée pour extruder une résine thermodurcissable à l'aide d'un moule de fabrication de tubes (8) pour fabriquer un premier tube (14) ;
un dispositif de pulvérisation d'agent de démoulage (22) disposé à l'extrémité arrière du moule de fabrication de tubes (8) et configuré pour appliquer alternativement un agent de démoulage sur une partie prédéterminée sur une périphérie extérieure du premier tube (14) ;
un premier dispositif de chauffage (38) configuré pour durcir et sécher le premier tube (14) qui a traversé le dispositif de pulvérisation d'agent de démoulage (22) et l'agent de démoulage enduit ;
une seconde extrudeuse (40) configurée pour extruder et recouvrir une couche de revêtement (46) sur la périphérie extérieure du premier tube (14), qui a traversé le premier dispositif de chauffage (38), à l'aide d'un moule de revêtement (41) ; et
un second dispositif de chauffage (42) configuré pour durcir le premier tube (14) sur lequel la couche de revêtement a été formée,
dans lequel le dispositif de pulvérisation d'agent de démoulage (22) comprend :
une buse (24) pour la pulvérisation d'un agent de démoulage ; et
un film de restriction de pulvérisation (26) plié pour limiter une portée de pulvérisation d'un agent de démoulage qui est pulvérisé à travers la buse (24).

2. Dispositif selon la revendication 1, dans lequel le film de restriction de pulvérisation (26) comprend en outre un dispositif de collecte de résidus d'agent de démoulage (30), le dispositif de collecte de résidus d'agent de démoulage (30) comprenant :
un tuyau d'aspiration (32), à l'intérieur duquel est formé un conduit de tuyau ;
une pluralité de trous d'aspiration (34) formés sur des surfaces latérales intérieures du tuyau d'aspiration (32) pour aspirer une quantité résiduelle d'un agent de démoulage ; et
un tuyau (36) relié à une pompe à vide de manière à appliquer une pression négative au tuyau d'aspiration (32).

3. Dispositif selon la revendication 1, dans lequel le film de restriction de pulvérisation (26) comprend en outre un moyen de chauffage pour l'évaporation d'un agent de démoulage résiduel accumulé sur une partie prédéterminée du film de restriction de pulvérisation (26).

4. Procédé de fabrication d'un cathéter à ballonnet à l'aide d'une résine thermodurcissable et du dispositif selon une ou plusieurs des revendications précédentes, le procédé comprenant :
a) l'extrusion de la résine thermodurcissable pour fabriquer un premier tube (14), sur une périphérie extérieure duquel une lumière de gonflage (16) est formée dans une direction longitudinale, dans lequel une zone de pulvérisation et une zone sans pulvérisation sont divisées par un film de restriction de pulvérisation (26) lors de la pulvérisation de l'agent de démoulage ;
b) un processus de pulvérisation d'un agent de démoulage d'une manière sans contact pour appliquer alternativement l'agent de démoulage à des sections prédéterminées sur la périphérie extérieure du premier tube (14) ;
c) le durcissement thermique [[a]] de la résine et le séchage de l'agent de démoulage à l'aide d'un appareil de chauffage ; et
d) le revêtement d'une partie extérieure du premier tube (14) avec un matériau pour ballonnet afin de fabriquer un tube de cathéter à ballonnet (50), dans lequel un ballonnet est formé de manière répétée, dans une direction longitudinale.

5. Procédé selon la revendication 4, comprenant en outre : l'aspiration périodique d'un résidu de l'agent de démoulage pulvérisé à travers des trous d'aspiration (34) formés dans le film de restriction de pulvérisation (26).

6. Procédé selon la revendication 4, comprenant en outre : l'installation d'un fil chauffant au niveau du film de restriction de pulvérisation (26) pour faire évaporer un résidu accumulé de l'agent de démoulage.
